# EUROPEAN PATENT APPLICATION

(11) **EP 3 733 850 A1**
(43) Date of publication of application: **04.11.2020**
(21) Application number: 18894129.8
(22) Date of filing: 28.12.2018
(51) Int. Cl.: C12N 15/113, C12N 15/63, C12N 5/0783

(54) **CHIMERIC PROMOTER WITH HIGH TRANSCRIPTIONAL ACTIVITY IN T-CELLS**

(30) Priority: 29.12.2017 CN 201711476630
(71) Applicant: Shanghai Cell Therapy Group Co., Ltd, Jiading District Anting Town Shanghai 201805 (CN)
(72) Inventor: QIAN, Qijun, Shanghai 201805 (CN); JIN, Huajun, Shanghai 201805 (CN); WANG, Chao, Shanghai 201805 (CN); HE,Zhou, Shanghai 201805 (CN); LI, Linfang, Shanghai 201805 (CN); SUN, Juanjuan, Shanghai 201805 (CN); HUANG, Chen, Shanghai 201805 (CN)
(74) Representative: Cabinet Beau de Loménie
(86) International application number: PCT/CN2018/124687
(87) International publication number: WO 2019/129175

(57) **Abstract**

Provided is a chimeric promoter with a high transcriptional activity in T-cells. The chimeric promoter consists of an enhancer sDTS and a promoter operably linked to the enhancer. The sequence of the enhancer is as shown in SEQ ID NO: 1. The enhancer is located at the 5' or 3' terminus of the promoter. Also provided are nucleic acid constructs, vectors and T-cells comprising the chimeric promoter. The chimeric promoter can drive the efficient expression of exogenous genes in T-cells.

Sequence listing

<110>  SHANGHAI CELL THERAPY RESEARCH INSTITUTE
       SHANGHAI CELL THERAPY GROUP CO., LTD

<120>  Chimeric promoter with high transcriptional activity in T-cells

<130>  17A173

<150>  CN 201711476630.6
<151>  2017-12-29

<160>  12

<170>  PatentIn version 3.5

<210>  1
<211>  144
<212>  DNA
<213>  Artificial Sequence

<220>
<223>  Description of Artificial Sequence: Nucleotide sequence of sDTS

<400>  1
ggtgtggaaa gtccccaggc tccccagcag gcagaagtat gcaaagcatg catctcaatt      60
agtcagcaac caggtgtgga aagtccccag gctccccagc aggcagaagt atgcaaagca     120
tgcatctcaa ttagtcagca acca                                            144

<210>  2
<211>  211
<212>  DNA
<213>  Artificial Sequence

<220>
<223>  Description of Artificial Sequence: Nucleotide sequence of sDTS-LTR

<400>  2
ggtgtggaaa gtccccaggc tccccagcag gcagaagtat gcaaagcatg catctcaatt      60
agtcagcaac caggtgtgga aagtccccag gctccccagc aggcagaagt atgcaaagca     120
tgcatctcaa ttagtcagca accattaacc ctagaaagat aatcatattg tgacgtacgt     180
taaagataat catgcgtaaa attgacgcat g                                    211

<210>  3
<211>  545
<212>  DNA
<213>  Artificial Sequence

<220>
<223>  Description of Artificial Sequence: Nucleotide sequence of EF1alpha
promoter

<400>  3
aggatctgcg atcgctccgg tgcccgtcag tgggcagagc gcacatcgcc cacagtcccc      60
gagaagttgg ggggaggggt cggcaattga acgggtgcct agagaaggtg gcgcggggta     120
aactgggaaa gtgatgtcgt gtactggctc cgcctttttc ccgagggtgg gggagaaccg     180
tatataagtg cagtagtcgc cgtgaacgtt ctttttcgca acgggtttgc cgccagaaca     240
cagctgaagc ttcgaggggc tcgcatctct ccttcacgcg cccgccgccc tacctgaggc     300
cgccatccac gccggttgag tcgcgttctg ccgcctcccg cctgtggtgc ctcctgaact     360
gcgtccgccg tctaggtaag tttaaagctc aggtcgagac cgggcctttg tccggcgctc     420
ccttggagcc tacctagact cagccggctc tccacgcttt gcctgaccct gcttgctcaa     480
ctctacgtct ttgtttcgtt ttctgttctg cgccgttaca gatccaagct gtgaccggcg     540
cctac                                                                 545

<210>  4
<211>  584
<212>  DNA
<213>  Artificial Sequence

<220>
<223>  Description of Artificial Sequence: Nucleotide sequence of hCMV
promoter

<400>  4
gacattgatt attgactagt tattaatagt aatcaattac ggggtcatta gttcatagcc      60
catatatgga gttccgcgtt acataactta cggtaaatgg cccgcctggc tgaccgccca     120
acgacccccg cccattgacg tcaataatga cgtatgttcc catagtaacg ccaataggga     180
ctttccattg acgtcaatgg gtggagtatt tacggtaaac tgcccacttg gcagtacatc     240
aagtgtatca tatgccaagt acgcccccta ttgacgtcaa tgacggtaaa tggcccgcct     300
ggcattatgc ccagtacatg accttatggg actttcctac ttggcagtac atctacgtat     360
tagtcatcgc tattaccatg gtgatgcggt tttggcagta catcaatggg cgtggatagc     420
ggtttgactc acggggattt ccaagtctcc accccattga cgtcaatggg agtttgtttt     480
ggcaccaaaa tcaacgggac tttccaaaat gtcgtaacaa ctccgcccca ttgacgcaaa     540
tgggcggtag gcgtgtacgg tgggaggtct atataagcag agct                      584

<210>  5
<211>  378
<212>  DNA
<213>  Artificial Sequence

<220>
<223>  Description of Artificial Sequence: Nucleotide sequence of beta-Actin
promoter

<400>  5
tcgaggtgag ccccacgttc tgcttcactc tccccatctc ccccccctcc ccacccccaa      60
ttttgtattt atttattttt taattatttt gtgcagcgat gggggcgggg gggggggggg     120
gcgcgcgcca ggcggggcgg ggcggggcga ggggcggggc ggggcgaggc ggagaggtgc     180
ggcggcagcc aatcagagcg gcgcgctccg aaagtttcct tttatggcga ggcggcggcg     240
gcggcggccc tataaaaagc gaagcgcgcg gcgggcggga gtcgctgcgc gctgccttcg     300
ccccgtgccc cgctccgccg ccgcctcgcg ccgcccgccc cggctctgac tgaccgcgtt     360
actaaaacag gtaagtcc                                                   378

<210>  6
<211>  600
<212>  DNA
<213>  Artificial Sequence

<220>
<223>  Description of Artificial Sequence: Nucleotide sequence of
nanoluciferase

<400>  6
atgaactcct tctccacaag cgccttcggt ccagttgcct tctccctggg cctgctcctg      60
gtgttgcctg ctgccttccc tgccccagtc ttcacactcg aagatttcgt tggggactgg     120
cgacagacag ccggctacaa cctggaccaa gtccttgaac agggaggtgt gtccagtttg     180
tttcagaatc tcggggtgtc cgtaactccg atccaaagga ttgtcctgag cggtgaaaat     240
gggctgaaga tcgacatcca tgtcatcatc ccgtatgaag gtctgagcgg cgaccaaatg     300
ggccagatcg aaaaaatttt taaggtggtg taccctgtgg atgatcatca ctttaaggtg     360
atcctgcact atggcacact ggtaatcgac ggggttacgc cgaacatgat cgactatttc     420
ggacggccgt atgaaggcat cgccgtgttc gacggcaaaa agatcactgt aacagggacc     480
ctgtggaacg gcaacaaaat tatcgacgag cgcctgatca accccgacgg ctccctgctg     540
ttccgagtaa ccatcaacgg agtgaccggc tggcggctgt gcgaacgcat tctggcgtaa     600

<210>  7
<211>  199

<212>  PRT
<213>  Artificial Sequence

<220>
<223>  Description of Artificial Sequence: Amino acid sequence of
nanoluciferase

<400>  7
Met Asn Ser Phe Ser Thr Ser Ala Phe Gly Pro Val Ala Phe Ser Leu
1               5                   10                  15
Gly Leu Leu Leu Val Leu Pro Ala Ala Phe Pro Ala Pro Val Phe Thr
            20                  25                  30
Leu Glu Asp Phe Val Gly Asp Trp Arg Gln Thr Ala Gly Tyr Asn Leu
        35                  40                  45
Asp Gln Val Leu Glu Gln Gly Gly Val Ser Ser Leu Phe Gln Asn Leu
    50                  55                  60
Gly Val Ser Val Thr Pro Ile Gln Arg Ile Val Leu Ser Gly Glu Asn
65                  70                  75                  80
Gly Leu Lys Ile Asp Ile His Val Ile Ile Pro Tyr Glu Gly Leu Ser
                85                  90                  95
Gly Asp Gln Met Gly Gln Ile Glu Lys Ile Phe Lys Val Val Tyr Pro
            100                 105                 110
Val Asp Asp His His Phe Lys Val Ile Leu His Tyr Gly Thr Leu Val
        115                 120                 125
Ile Asp Gly Val Thr Pro Asn Met Ile Asp Tyr Phe Gly Arg Pro Tyr
    130                 135                 140
Glu Gly Ile Ala Val Phe Asp Gly Lys Lys Ile Thr Val Thr Gly Thr
145                 150                 155                 160
Leu Trp Asn Gly Asn Lys Ile Ile Asp Glu Arg Leu Ile Asn Pro Asp
                165                 170                 175
Gly Ser Leu Leu Phe Arg Val Thr Ile Asn Gly Val Thr Gly Trp Arg
            180                 185                 190
Leu Cys Glu Arg Ile Leu Ala
        195

<210>  8
<211>  720
<212>  DNA
<213>  Artificial Sequence

<220>
<223>  Description of Artificial Sequence: Nucleotide sequence of EGFP

<400>  8
atggtgagca agggcgagga gctgttcacc ggggtggtgc ccatcctggt cgagctggac      60
ggcgacgtaa acggccacaa gttcagcgtg tccggcgagg gcgagggcga tgccacctac     120
ggcaagctga ccctgaagtt catctgcacc accggcaagc tgcccgtgcc ctggcccacc     180
ctcgtgacca ccctgaccta cggcgtgcag tgcttcagcc gctaccccga ccacatgaag     240
cagcacgact tcttcaagtc cgccatgccc gaaggctacg tccaggagcg caccatcttc     300
ttcaaggacg acggcaacta caagacccgc gccgaggtga agttcgaggg cgacaccctg     360
gtgaaccgca tcgagctgaa gggcatcgac ttcaaggagg acggcaacat cctggggcac     420
aagctggagt acaactacaa cagccacaac gtctatatca tggccgacaa gcagaagaac     480
ggcatcaagg tgaacttcaa gatccgccac aacatcgagg acggcagcgt gcagctcgcc     540
gaccactacc agcagaacac ccccatcggc gacggccccg tgctgctgcc cgacaaccac     600
tacctgagca cccagtccgc cctgagcaaa gaccccaacg agaagcgcga tcacatggtc     660
ctgctggagt tcgtgaccgc cgccgggatc actctcggca tggacgagct gtacaagtaa     720

<210>  9
<211>  239
<212>  PRT
<213>  Artificial Sequence

<220>

<223>  Description of Artificial Sequence: Amino acid sequence of EGFP

<400>  9
Met Val Ser Lys Gly Glu Glu Leu Phe Thr Gly Val Val Pro Ile Leu
1               5                   10                  15
Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Thr Tyr Gly Val Gln Cys Phe Ser Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Asn Ser His Asn Val Tyr Ile Met Ala Asp Lys Gln Lys Asn
145                 150                 155                 160
Gly Ile Lys Val Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Asn Glu Lys Arg Asp His Met Val Leu Leu Glu Phe
    210                 215                 220
Val Thr Ala Ala Gly Ile Thr Leu Gly Met Asp Glu Leu Tyr Lys
225                 230                 235

<210>  10
<211>  1488
<212>  DNA
<213>  Artificial Sequence

<220>
<223>  Description of Artificial Sequence: Nucleotide sequence of anti-PD-1
antibody

<400>  10
atggaagccc cagctcagct tctcttcctc ctgctactct ggctcccaga taccaccgga      60
gaaattgtgt tgacacagtc tccagccacc ctgtctttgt ctccagggga aagagccacc     120
ctctcctgca gggccagcaa aggtgtcagt acatctggct atagttattt gcactggtat     180
caacagaaac ctggccaggc tcccaggctc ctcatctatc ttgcatccta cctagaatct     240
ggcgtcccag ccaggttcag tggtagtggg tctgggacag acttcactct caccatcagc     300
agcctagagc ctgaagattt tgcagtttat tactgtcagc acagcaggga ccttccgctc     360
acgttcggcg gagggaccaa agtggagatc aaaggtggag gcggttcagg cggaggtggc     420
agcggcggtg gcgggtcgca ggtgcagctg gtgcagtccg gcgtggaggt gaagaagcct     480
ggcgcctccg tcaaggtgtc ctgtaaggcc tccggctaca ccttcaccaa ctactacatg     540
tactgggtgc ggcaggcccc aggccaggga ctggagtgga tgggcggcat caacccttcc     600
aacggcggca ccaacttcaa cgagaagttc aagaaccggg tgaccctgac caccgactcc     660
tccaccacaa ccgcctacat ggaactgaag tccctgcagt tcgacgacac cgccgtgtac     720
tactgcgcca ggcgggacta ccggttcgac atgggcttcg actactgggg ccagggcacc     780
accgtgaccg tgtcctccga gtccaaatat ggtcccccat gcccaccatg cccagcacct     840
gagttcgagg ggggaccatc agtcttcctg ttccccccaa aacccaagga cactctcatg     900
atctcccgga cccctgaggt cacgtgcgtg gtggtggacg tgagccagga agaccccgag     960
gtccagttca actggtacgt ggatggcgtg gaggtgcata atgccaagac aaagccgcgg    1020
gaggagcagt tccagagcac gtaccgtgtg gtcagcgtcc tcaccgtcct gcaccaggac    1080
tggctgaacg gcaaggagta caagtgcaag gtctccaaca aaggcctccc gtcctccatc    1140

gagaaaacca tctccaaagc caaagggcag ccccgagagc cacaggtgta caccctgccc 1200
ccatcccagg aggagatgac caagaaccag gtcagcctga cctgcctggt caaaggcttc 1260
taccccagcg acatcgccgt ggagtgggag agcaatgggc agccggagaa caactacaag 1320
accacgcctc ccgtgctgga ctccgacggc tccttcttcc tctacagcag gctaaccgtg 1380
gacaagagca ggtggcagga ggggaatgtc ttctcatgct ccgtgatgca tgaggctctg 1440
cacaaccact acacacagaa gagcctctcc ctgtctctgg gtaaatga 1488

<210> 11
<211> 495
<212> PRT
<213> Artificial Sequence

<220>
<223> Description of Artificial Sequence: Amino acid sequence of anti-PD-1
antibody

<400> 11
Met Glu Ala Pro Ala Gln Leu Leu Phe Leu Leu Leu Leu Trp Leu Pro
1               5                   10                  15
Asp Thr Thr Gly Glu Ile Val Leu Thr Gln Ser Pro Ala Thr Leu Ser
            20                  25                  30
Leu Ser Pro Gly Glu Arg Ala Thr Leu Ser Cys Arg Ala Ser Lys Gly
        35                  40                  45
Val Ser Thr Ser Gly Tyr Ser Tyr Leu His Trp Tyr Gln Gln Lys Pro
    50                  55                  60
Gly Gln Ala Pro Arg Leu Leu Ile Tyr Leu Ala Ser Tyr Leu Glu Ser
65                  70                  75                  80
Gly Val Pro Ala Arg Phe Ser Gly Ser Gly Ser Gly Thr Asp Phe Thr
                85                  90                  95
Leu Thr Ile Ser Ser Leu Glu Pro Glu Asp Phe Ala Val Tyr Tyr Cys
            100                 105                 110
Gln His Ser Arg Asp Leu Pro Leu Thr Phe Gly Gly Gly Thr Lys Val
        115                 120                 125
Glu Ile Lys Gly Gly Gly Gly Ser Gly Gly Gly Gly Ser Gly Gly Gly
    130                 135                 140
Gly Ser Gln Val Gln Leu Val Gln Ser Gly Val Glu Val Lys Lys Pro
145                 150                 155                 160
Gly Ala Ser Val Lys Val Ser Cys Lys Ala Ser Gly Tyr Thr Phe Thr
                165                 170                 175
Asn Tyr Tyr Met Tyr Trp Val Arg Gln Ala Pro Gly Gln Gly Leu Glu
            180                 185                 190
Trp Met Gly Gly Ile Asn Pro Ser Asn Gly Gly Thr Asn Phe Asn Glu
        195                 200                 205
Lys Phe Lys Asn Arg Val Thr Leu Thr Thr Asp Ser Ser Thr Thr Thr
    210                 215                 220
Ala Tyr Met Glu Leu Lys Ser Leu Gln Phe Asp Asp Thr Ala Val Tyr
225                 230                 235                 240
Tyr Cys Ala Arg Arg Asp Tyr Arg Phe Asp Met Gly Phe Asp Tyr Trp
                245                 250                 255
Gly Gln Gly Thr Thr Val Thr Val Ser Ser Glu Ser Lys Tyr Gly Pro
            260                 265                 270
Pro Cys Pro Pro Cys Pro Ala Pro Glu Phe Glu Gly Gly Pro Ser Val
        275                 280                 285
Phe Leu Phe Pro Pro Lys Pro Lys Asp Thr Leu Met Ile Ser Arg Thr
    290                 295                 300
Pro Glu Val Thr Cys Val Val Val Asp Val Ser Gln Glu Asp Pro Glu
305                 310                 315                 320
Val Gln Phe Asn Trp Tyr Val Asp Gly Val Glu Val His Asn Ala Lys
                325                 330                 335
Thr Lys Pro Arg Glu Glu Gln Phe Gln Ser Thr Tyr Arg Val Val Ser
            340                 345                 350
Val Leu Thr Val Leu His Gln Asp Trp Leu Asn Gly Lys Glu Tyr Lys
        355                 360                 365

Cys Lys Val Ser Asn Lys Gly Leu Pro Ser Ser Ile Glu Lys Thr Ile
    370                 375                 380
Ser Lys Ala Lys Gly Gln Pro Arg Glu Pro Gln Val Tyr Thr Leu Pro
385                 390                 395                 400
Pro Ser Gln Glu Glu Met Thr Lys Asn Gln Val Ser Leu Thr Cys Leu
                405                 410                 415
Val Lys Gly Phe Tyr Pro Ser Asp Ile Ala Val Glu Trp Glu Ser Asn
            420                 425                 430
Gly Gln Pro Glu Asn Asn Tyr Lys Thr Thr Pro Pro Val Leu Asp Ser
        435                 440                 445
Asp Gly Ser Phe Phe Leu Tyr Ser Arg Leu Thr Val Asp Lys Ser Arg
    450                 455                 460
Trp Gln Glu Gly Asn Val Phe Ser Cys Ser Val Met His Glu Ala Leu
465                 470                 475                 480
His Asn His Tyr Thr Gln Lys Ser Leu Ser Leu Ser Leu Gly Lys
                485                 490                 495

<210>  12
<211>  5037
<212>  DNA
<213>  Artificial Sequence

<220>
<223>  Description of Artificial Sequence: Nucleotide sequence of pNB338B-E
vector

<400>  12
ggcgcgcctt aaccctagaa agataatcat attgtgacgt acgttaaaga taatcatgcg      60
taaaattgac gcatgtctag aggtgtggaa agtccccagg ctccccagca ggcagaagta     120
tgcaaagcat gcatctcaat tagtcagcaa ccaggtgtgg aaagtcccca ggctccccag     180
caggcagaag tatgcaaagc atgcatctca attagtcagc aaccaaggat ctgcgatcgc     240
tccggtgccc gtcagtgggc agagcgcaca tcgcccacag tccccgagaa gttgggggga     300
ggggtcggca attgaacggg tgcctagaga aggtggcgcg gggtaaactg ggaaagtgat     360
gtcgtgtact ggctccgcct ttttcccgag ggtgggggag aaccgtatat aagtgcagta     420
gtcgccgtga acgttctttt tcgcaacggg tttgccgcca gaacacagct gaagcttcga     480
ggggctcgca tctctccttc acgcgcccgc cgccctacct gaggccgcca tccacgccgg     540
ttgagtcgcg ttctgccgcc tcccgcctgt ggtgcctcct gaactgcgtc cgccgtctag     600
gtaagtttaa agctcaggtc gagaccgggc ctttgtccgg cgctcccttg gagcctacct     660
agactcagcc ggctctccac gctttgcctg accctgcttg ctcaactcta cgtctttgtt     720
tcgttttctg ttctgcgccg ttacagatcc aagctgtgac cggcgcctac gaattcgtcg     780
accagacatg ataagataca ttgatgagtt tggacaaacc acaactagaa tgcagtgaaa     840
aaaatgcttt atttgtgaaa tttgtgatgc tattgcttta tttgtaacca ttataagctg     900
caataaacaa gttaacaaca acaattgcat tcattttatg tttcaggttc agggggaggt     960
gtgggaggtt ttttaaagca agtaaaacct ctacaaatgt ggtagcatgc gtcaatttta    1020
cgcagactat ctttctaggg ttaaatcgat ttagaagcag ctctggcaca tgtcgatgtt    1080
gtgctcgcgg cagatcacct tcttgcactt cttgcagctg gcgctggcct tgcggcggat    1140
cttgctgggg cagtaggtgc agtaggtgcg cttcttcatc acgggctcct cggtgctgtc    1200
gtcgctggtg ccgggcacct ccttgggcag gatgttgctg atgttgtcgc gcaggtagcg    1260
cttcagggtg ggggcctcca ggcgcttgcg catgaagctg ctggtcaggc ccatgtacag    1320
gttgcgcatg aacttcttgc ggctctgcac cttctcgccc ttgctgctca cgttgtggct    1380
gtagatgatg aagctgttga tgcaggcgat gttgatcatg ccgtacagca gggccatggg    1440
ccagcggttg gtcttgcggc tgcaggtcat cacgctgcac atctggtcca gggtgtccac    1500
gccgcccttg gtctggttgt agtacatcac catctggggc ttgccggtgc tctcgttgat    1560
gctggcgtcc tcgtcgcagc tgctcagcag gtacaccatc ttggcgggct tgggcttgta    1620
gctcaccagg gtcagggggc cgtcgaagca gaacatgctg gtgcccacgg ggcggctgcg    1680
gctgttcttc agcacctcgg ggatctcgcg cttgttgctg cgcacggtgc ccacgatggt    1740
cagcttgtag ggctcctgca gcaggttctt ggccaggggg atgctggtga accagttgtc    1800
gcaggtgatg ttgcggcagc tgccgtgcac gggcttgctc agctccttca cgtagtactc    1860
gcccaggggc acgccgttgg tctgggtgcc gcggcccagg tagggcatgc cgttgatcat    1920
gtacttggtg ccgctgtcgc acatcatcag gatcttgatg ccgtacttgc tgggcttgtt    1980
ggggatgtac acgcggaagg ggcagcggcc gcggaagccc agcagctgct cgtcgatggt    2040
caggtgggcg ccgggggtgt agttctggat gcactggtgg atgaacaggt cccagatctt    2100
gcgcacgggg gtgaacacgt cgttctcgcg cagggtgggg cggatgctct tgtcgtccat    2160

gcgcaggcag cggatcagga agtcgaagcg gtcgcggctc atcacgctca cgtacaccat 2220
gctcaggctg cggtcgaaca ggtcgtcggt gctcatgtgg ttgtccttgc gcacggcggt 2280
catcaccagg atgccgaaga aggcgtagat ctcgtcctcg ttggtgtcgc ggaaggtggc 2340
gctggtcatg ctctcgcggc gcttcaggct gatctcggcg ttggtccact tcacgatctc 2400
gctgatgatc tcgtcggtga agaacagctt gaagcacagc agggggtcgt agatgttgcg 2460
gcacatgcgg gtggggccgc gctggctgcg cacgatgttc agggcgctca cgcggctgcg 2520
gcgggtgggc ttgctggtgc tccagcagtg cttgttcttg ccgcggatgg tgcgctgggg 2580
cagggtcagg atgcggttgc tggccaggct gctgccgggc tgctcgatca cgttctgctc 2640
gtccaggatc tcgctgccgc tgctggtggg ctgcacctcg tgcacctcgt cgatgaaggc 2700
ctcctcggtg tcgctctgca cgtcgtcctc gctcacgtgg tcgctcacct cgctgtcgct 2760
gtcctcgccc accagctcgt cgtcgctctg cagcagggcg ctcaggatgt gctcgtcgtc 2820
caggctgctg ccgtccaact tgaccctctt ggcagcaggg cccatggtgg caagcttgat 2880
ctgcgttcta cggtggtcag accgaagact gcgacggtac cgacgctggt cgcgcctctt 2940
atacccacgt agaacgcagc tcagccaata gaatgagtgc caatatggaa tttccagggg 3000
aaaaccgggg cggtgttacg ttttggctgc cctttcactt cccattgacg tgtattggct 3060
cgagaacggt actttcccat taatcagcta tgggaaagta ccgtttaaag gtcacgttgc 3120
attagtttca atagcccatt gacgtcaatg gtgggaaagt acatggcgtt ttaatttaat 3180
ggctggaaaa acccaatgac tcacccctat tgaccttatg tacgtgccaa taatgggaaa 3240
aacccattga ctcaccccct attgaccttt tgtactgggc aaaacccaat ggaaagtccc 3300
tattgactca gtgtacttgg ctccaatggg actttcctgt tgattcaccc ctattgacct 3360
tatgtactgg gcaaaaccca ttggaaagtc cctaatgact cagtatattt aattaagagg 3420
gggagaccaa agggcgagac gttaaggcct cacgtgacat gtgagcaaaa ggccagcaaa 3480
aggccaggaa ccgtaaaaag gccgcgttgc tggcgttttt ccataggctc cgcccccctg 3540
acgagcatca caaaaatcga cgctcaagtc agaggtggcg aaacccgaca ggactataaa 3600
gataccaggc gtttccccct ggaagctccc tcgtgcgctc tcctgttccg accctgccgc 3660
ttacgggata cctgtccgcc tttctccctt cgggaagcgt ggcgctttct catagctcac 3720
gctgtaggta tctcagttcg gtgtaggtcg ttcgctccaa gctgggctgt gtgcacgaac 3780
cccccgttca gcccgaccgc tgcgccttat ccggtaacta tcgtcttgag tccaacccgg 3840
taagacacga cttatcgcca ctggcagcag ccactggtaa caggattagc agagcgaggt 3900
atgtaggcgg tgctacagag ttcttgaagt ggtggcctaa ctacggctac actagaagaa 3960
cagtatttgg tatctgcgct ctgctgaagc cagttacctt cggaaaaaga gttggtagct 4020
cttgatccgg caaacaaacc accgctggta gcggtggttt ttttgtttgc aagcagcaga 4080
ttacgcgcag aaaaaaagga tctcaagaag atcctttgat cttttctacg gggtctgacg 4140
ctcagtggaa cgaaaactca cgttaaggga ttttggtcat gccgtctcag aagaactcgt 4200
caagaaggcg atagaaggcg atgcgctgcg aatcgggagc ggcgataccg taaagcacga 4260
ggaagcggtc agcccattcg ccgccaagct cttcagcaat atcacgggta gccaacgcta 4320
tgtcctgata gcggtccgcc acacccagcc ggccacagtc gatgaatcca gaaaagcggc 4380
cattttccac catgatattc ggcaagcagg catcgccatg ggtcacgacg agatcctcgc 4440
cgtcgggcat gctcgccttg agcctggcga acagttcggc tggcgcgagc ccctgatgct 4500
cttcgtccag atcatcctga tcgacaagac cggcttccat ccgagtacgt gctctctcga 4560
tgcgatgttt cgcttggtgg tcgaatgggc aggtagccgg atcaagcgta tgcagccgcc 4620
gcattgcatc agccatgatg gatactttct cggcaggagc aaggtgagat gacaggagat 4680
cctgccccgg cacttcgccc aatagcagcc agtcccttcc cgcttcagtg acaacgtcga 4740
gtacagctgc gcaaggaacg cccgtcgtgg ccagccacga tagccgcgct gcctcgtctt 4800
gcagttcatt cagggcaccg gacaggtcgg tcttgacaaa aagaaccggg cgcccctgcg 4860
ctgacagccg gaacacggcg gcatcagagc agccgattgt ctgttgtgcc cagtcatagc 4920
cgaatagcct ctccacccaa gcggccggag aacctgcgtg caatccatct tgttcaatca 4980
taatattatt gaagcattta tcagggttcg tctcgtcccg gtctcctccc atgcatg 5037

## Description

### Technical Field

The disclosure belongs to the field of molecular biology, and relates to a chimeric promoter with high activity in T cells and uses thereof. The promoter is a synthetic chimeric promoter and has high activity in T cells. The disclosure also relates to recombinant vectors and recombinant viruses comprising the promoter and uses of the promoter in transgenic T cell therapy through controlling exogenous gene.

### Background

Cancer has been studied for a hundred years, but so far it has not been completely conquered. With the emergence of surgical treatment, chemotherapy, radiotherapy and other treatments, the survival rate of cancer patients has been greatly improved, but these therapies are not enough to save tens of millions of cancer patients in the world. With the vigorous development of biotechnology, biotherapy, as a new treatment method, came into being, and has become the fourth largest therapy in the field of cancer treatment.

Biotherapy includes a variety of models, such as immunotherapy (cells, cytokines, antibodies, vaccines, etc.), gene therapy, regulatory angiogenesis therapy, small molecule targeted drugs and stem cell and tissue engineering regenerative medicine. Among them, cancer immunotherapy has been widely concerned by academic institutions and pharmaceutical companies since it was selected as one of the six most noteworthy scientific fields of Science in 2013.

Cancer immunotherapy mainly includes adoptive cell therapy, immunomodulators, tumor vaccines and immune checkpoint blockers. Among them, immune checkpoint blockers are effective in the treatment of many cancers, especially malignant and chemoresistant cancers. Four kinds of immune checkpoint blockers have been approved by FDA. In addition, in the field of adoptive cell therapy, especially the new generation CAR-T cell therapy technology has shown amazing therapeutic effect for blood tumors, including leukemia and lymphoma. At present, the preparation of CAR-T cells is mainly based on viral and non-viral expression systems, and viral vectors are likely to cause cell replication and insertional mutagenesis, which may lead to biosafety problems. In 2013, Cooper Laboratory of MD Anderson Cancer Center used a non-viral transfection method, namely sleeping beauty (SB) transposon system, to modify gene to obtain CD19 antigen-specific CAR-T cells.

Promoter is a component of a gene, usually located upstream of the 5'end of a structural gene. It is a DNA sequence recognized and bound by RNA polymerase and from which the transcription starts. Promoter is one of the important factors that affect the transgenic expression efficiency, and choosing a highly efficient promoter is the key to efficiently expression of an exogenous gene.

According to their transcriptional patterns, promoters can be divided into three types: constitutive promoters, tissue or organ specific promoters and inducible promoters.

Constitutive promoters refer to promoters that under their regulation there is no significant difference in gene expression in different tissues, organs and development stages, and are therefore called constitutive promoters. Constitutive promoters commonly used in mammals include those from viruses: murine or human cytomegalovirus (CMV) promoters (mCMV and hCMV, respectively), monkey vacuolar virus SV40 promoter; those from natural human genome: EF1α promoter, ubiquitin promoter (Ubi for short), β-actin promoter, PGK-1 promoter, Rosa26 promoter, HSP70 promoter, GAPDH promoter, eIF4A1 promoter, Egr1 promoter, FerH promoter, SM22α promoter, Endothelin-1 promoter, etc.

In tumor immunotherapy, it is very important to maintain the highly efficient and stable expression of exogenous genes. However, although the transient expression activity of some constitutive promoters from viruses is high (such as CMV promoter), such expression is easy to be stop due to epigenetic modification; while some natural constitutive promoters from human or tumor specific promoters are stable in expression, but their expression activity is relatively low, which is difficult to meet the needs of immunotherapy. Therefore, the researchers designed and constructed a series of artificial chimeric promoters, which comprised some cis-acting elements, mainly including promoter core sequences which could stabilize expression, and upstream enhancers or downstream introns that can enhance the expression efficiency. A Represented artificial chimeric promoters is the chimeric promoter CAG (including human CMV enhancer-chicken β-actin promoter-rabbit β-globin intron), which has been widely used in the expression of exogenous genes.

Enhancer refers to the DNA sequence that increases the transcription frequency of genes linked with it. Enhancers increase the transcription of downstream genes through promoters. Effective enhancers can be located at the 5' end, at the 3' end or in an intron of a gene. Enhancers have significant effect, which generally can increase the gene transcription frequency by 10∼200-folds, some even up to thousands-folds.

### SUMMARY

To solve the technical problem that the transcription intensity of the promoter in the expression vector is not high enough in the prior art, the inventor designed and constructed a series of novel chimeric promoters through a lot of experiments and creative labor, and screened out a group of promoters that can express efficiently in T cells. The promoter is especially suitable for driving the high expression of exogenous genes (such as full-length antibody genes) in T cells.

Therefore, one aspect of the disclosure provides an enhanced promoter sequence (i.e. chimeric promoter), the enhanced promoter sequence consisting of an enhancer sDTS and a promoter operably linked to the enhancer, wherein the sequence of the enhancer sDTS is as shown in SEQ ID NO: 1.

In one or more embodiments, the promoter is selected from the group consisting of: an EF1α promoter, a PGK promoter, a β-actin promoter, an hCMV promoter, an EEF2 promoter, a CAG promoter, a U6 promoter and a SV40 promoter.

In one or more embodiments, the promoter is an EF1α promoter, an hCMV promoter or a β-actin promoter, preferably an EF1α promoter.

In one or more embodiments, the enhancer is located at the 5 'end or 3' end of the promoter, preferably located at the 5' end of the promoter.

In one or more embodiments, the nucleic acid sequence of the enhanced promoter sequence consists of the nucleic acid sequence as shown in SEQ ID NO: 1 and the nucleic acid sequence as shown in SEQ ID NO: 3.

The disclosure also provides a nucleotide construct comprising the enhanced promoter sequence described herein.

In one or more embodiments, the nucleic acid construct is an expression cassette, which sequentially comprises, from the 5' end to the 3' end, the enhanced promoter sequence, the coding sequence of a protein of interest and a transcription terminator, that are operably linked.

The disclosure also provides a vector comprising the enhanced promoter sequence described herein.

In one or more embodiments, the vector is a eukaryotic expression vector, selected from the group consisting of: a transient expression vector, a virus expression vector and a transposition vector.

In one or more embodiments, the vector is a vector for integrating the expression cassette of a gene of interest into the genome of a host cell, preferably is a transposition vector.

In one or more embodiments, the transposition vector comprises a transposable element selected from the group consisting of: piggyback, sleeping beauty, frog prince, Tn5 and Ty.

In one or more embodiments, the transposition vector sequentially comprises the enhanced promoter sequence, one or more restriction sites or optional coding sequence(s) of interest and a transcription termination sequence, that are operably linked between the 5' LTR and the 3' LTR.

In one or more embodiments, a coding sequence of a transposase and its promoter sequence are operably linked at the 3' end of the 3' LTR.

In one or more embodiments, the vector also comprises one or more selectable markers.

In one or more embodiments, the coding sequence of interest is a coding sequence of an antibody.

In one or more embodiments, the antibody is selected from the group consisting of: a PD-1 antibody, a PD-L1 antibody, a CTLA4 antibody, a CD40 antibody, a CD40L antibody, a CD47 antibody, a CD137 antibody, a CD28 antibody, a CD27 antibody, a OX40 antibody, a DNAM-1 antibody, a GITR antibody, a ICSOS antibody, a 2B4 antibody, a CD160 antibody, a TIM3 antibody, a LAG3 antibody, a BTLA antibody and a TIGIT antibody; preferably a PD-1 single-chain antibody.

In one or more embodiments, the restriction site is selected from the group consisting of: AscI restriction site, XbaI restriction site, PvuI restriction site, EcoRI restriction site and SalI restriction site.

In one or more embodiments, the nucleic acid sequence of the vector is as shown in SEQ ID NO: 12.

The disclosure also provides the use of the enhanced promoter sequence described herein in driving the expression of an exogenous gene (such as a full-length antibody gene) in a T cell.

### BRIEF DESCRIPTION OF DRAWINGS

Figure 1: A schematic diagram of the structures of the expression vectors carrying the nucleotide sequence of each promoter.
Figure 2A-2B: The fluorescence intensities (2A) and positive rates (2B) of the expressions of pNB328-E-EGFP, pNB338A-E-EGFP and pNB338B-E-EGFP plasmids in T cell lines.
Figure 3: The Nluc fluorescence values of the expressions of pNB328-E-Nluc, pNB338B-E-Nluc, pNB328-hC-Nluc, pNB338B-hC-Nluc, pNB328-β-Nluc and pNB338B-β-Nluc plasmids in T cell lines.
Figure 4: A comparison of the expressions of PD-1 antibodies expressed by pNB328-E-m279V, pNB338B-E-m279V, pNB328-hC-m279V, pNB338B-hC-m279V, pNB328-β-m279V and pNB338B-β-m279V plasmids.
Figure 5: The expressions of EGFP protein in CHO cells mediated by EFla and sDTS+EF1a promoters.

### DETAILED DESCRIPTION

Some terms involved in the disclosure are explained below. It should be understood that, unless otherwise defined herein, terms used herein have meanings generally recognized in the art.

In the present disclosure, the term "expression cassette" refers to the complete elements required to express a gene, including operably linked promoter(s) and gene coding sequence(s).

The term "coding sequence" refers to a part of a nucleic acid sequence that directly determines the amino acid sequence of its protein product. The boundaries of a coding sequence are usually determined by a ribosome binding site closely adjacent to the upstream of the open reading frame at mRNA 5' end (for prokaryotic cells) and a transcription termination sequence closely adjacent to the downstream of the open reading frame at mRNA 3' end. The coding sequence can include, but is not limited to, DNA, cDNA and recombinant nucleic acid sequences.

The term "single-chain antibody" (scFv) refers to an antibody fragment formed by linking the amino acid sequences of antibody light chain variable region (V_{L} region) and the amino acid sequences of heavy chain variable region (V_{H} region) with hinge, which has antigen-binding ability. In some embodiments, the single-chain antibody (scFv) of interest is from an antibody of interest. The antibodies of interest may be human antibodies, including human-murine chimeric antibodies and humanized antibodies. The antibody may be a secretory antibody or a membrane-anchored antibody.

The term "operably linked" or "in operably linkage" refers to DNA regulatory sequences (such as enhancers, promoters, etc) linked to the coding sequence of a protein of interest in a manner that allows the expression of the coding sequence of the protein of interest.

The disclosure improves the expression intensity of the gene driven by a promoter by modifying the promoter activity, so as to obtain a high-efficiency expression of an exogenous gene, especially the full-length antibody genes in T cells.

The enhanced promoter sequence of the disclosure consists of an enhancer sDTS and a promoter operably linked to the enhancer, wherein the sequence of the enhancer sDTS is as shown in SEQ ID NO: 1.

The promoter in the enhanced promoter sequence of the disclosure can be any promoter sequence conventionally used in the art to express a exogenous gene in T cells, including but not limited to a EF1α promoter, a PGK promoter, a β-actin promoter, a hCMV promoter, a EEF2 promoter, a CAG promoter, a U6 promoter and a SV40 promoter; preferably, the promoter is a EF1α promoter, a hCMV promoter and a β-actin promoter, more preferably a EF1α promoter. The nucleotide sequence of an exemplary EF1α promoter can be as shown in SEQ ID NO: 3; the nucleotide sequence of an exemplary hCMV promoter can be as shown in SEQ ID NO: 4; the nucleotide sequence of an exemplary β-actin promoter can be as shown in SEQ ID NO: 5.

In some embodiments, the enhanced promoter sequence of the disclosure consists of SEQ ID NO: 1 and SEQ ID NO: 3, 4 or 5, preferably consists of SEQ ID NO: 1 and SEQ ID NO: 3.

The disclosure includes nucleic acid construct comprising the enhanced promoter sequence described herein.

In some embodiments, the nucleic acid is an expression cassette comprising the enhanced promoter sequence described herein and a coding sequence of a protein of interest. The expression cassette usually comprises a transcription termination sequence (i.e. transcription terminator), which is a sequence recognized by host cells to terminate transcription. The transcription termination sequence is operably linked to the 3' end of the coding sequence described herein. Any terminator that functions in the selected host cell can be used in the present disclosure, including but not limited to the SV40 polyA transcription termination sequence.

In some embodiments, the nucleic acid construct is a vector. The vector usually includes but is not limited to a plasmid, a phage particle, a phage derivative, an animal virus and a cosmid. The vector may be an expression vector, including a transient expression vector, a viral expression vector and a transposition vector. The vector is preferably a eukaryotic expression vector. Viruses that can be used as vectors include but are not limited to retroviruses, adenoviruses, adeno-associated viruses, herpesviruses and lentiviruses.

Generally, a suitable vector comprises at least one origin of replication that function in the host cell, convenient restriction site(s), and one or more selectable markers.

The restriction site includes but is not limited to an AscI restriction site, an XbaI restriction site, a PvuI restriction site, an EcoRI restriction site and a SalI restriction site. Generally, some of the restriction sites in the vector is located between the enhanced promoter sequence of the present disclosure and the transcription termination sequence, which is used to cut the vector there and insert a coding sequence of a protein of interest, so that the coding sequence can be operably linked to the enhanced promoter sequence of the disclosure and the transcription termination sequence.

The selectable markers include any one or both of selectable marker genes and reporter genes, so as to identify and select the expressing cells from the cell population infected by virus vector(s). Useful selectable marker genes include, for example, antibiotic resistance genes, such as neo and the like. Suitable reporter genes can include a gene encoding luciferase, β-galactosidase, chloramphenicol acetyltransferase, secretory alkaline phosphatase or green fluorescent protein.

In some embodiments, the vector is a vector for integrating an expression cassette of a gene of interest into the genome of a host cell, preferably is a transposition vector. In some embodiments, the transposition vector is a eukaryotic expression vector comprising a transposable element selected from the group consisting of: piggyback, sleeping beauty, frog prince, Tn5 and Ty. Such transposition vector comprises the 5' inverted terminal repeat (5' LTR) of the corresponding transposon and the 3' inverted terminal repeat (3' LTR) of the corresponding transposon. The transposase can be a transposase from piggybac, sleeping beauty, frog prince, Tn5 or Ty transposon system. When transposases from different transposon systems are used, the sequences of the 5' LTR and 3' LTR in the vector are changed accordingly into the sequences suitable for the transposon system, which can be easily determined by those skilled in the art.

In some embodiments, the transposase is a transposase from piggyback transposon system. Therefore, in these embodiments, the transposon 5' inverted terminal repeat and 3' inverted terminal repeat are the 5' inverted terminal repeat and 3' inverted terminal repeat of piggybac transposon, respectively. In some embodiments, the transposon 5' inverted terminal repeat is as shown in SEQ ID NO: 1 of CN201510638974.7 (the contents of which are incorporated herein by reference). In some embodiments, the transposon 3' inverted terminal repeat is as shown in SEQ ID NO: 4 of CN201510638974.7. In some embodiments, the piggyback transposase is a transposase comprising c-myc nuclear positioning signal coding sequence. In some embodiments, the coding sequence of the piggybac transposase is as shown in SEQ ID NO: 5 of CN201510638974.7.

Promoters of the transposase coding sequences may be various promoters known in the art for controlling the expression of the transposase coding sequences. In some embodiments, CMV promoter is used to control the expression of the transposase coding sequence. The sequence of CMV promoter can be as shown in SEQ ID NO: 6 of CN201510638974.7.

In some embodiments, the pNB328 vector disclosed in CN201510638974.7 is used as the skeleton of the vector of the disclosure, but the EF1α promoter contained in the vector is replaced by the enhanced promoter sequence described herein.

In some embodiments, the vector of the disclosure is an empty vector, that is, it does not comprise the coding sequence of a protein of interest. Generally, such empty vectors sequentially comprise the enhanced promoter sequence described herein, one or more restriction endonuclease sites and a transcription termination sequence, which are used to link the coding sequence of a protein of interest between the enhanced promoter sequence and the transcription termination sequence by enzymatic digestion. In some embodiments, the vector of the disclosure is a vector in which the coding sequence of a protein of interest is inserted between the enhanced promoter sequence described herein and a transcription termination sequence, preferably is a transposition vector. Such a vector sequentially comprises the enhanced promoter sequence described herein, the coding sequence of the protein of interest and the transcription termination sequence between the 5' LTR and 3' LTR, preferably also comprises a coding sequence of a transposase and its promoter sequence at the 3' end of the 3' LTR.

The disclosure also includes complementary sequences of each nucleotide sequence described herein. The polynucleotide sequences herein can be in the form of DNA or RNA.

The nucleotide sequences described here can generally be obtained by PCR amplification. Specifically, sequences of interest can be amplified by use of primers designed based on the nucleotide sequences disclosed herein, and commercially available cDNA libraries or cDNA libraries prepared according to conventional methods known to those skilled in the art as templates. When the sequence is long, it is often necessary to carry out two or more PCR amplifications, and then the amplified fragments are spliced together in the correct order. In some embodiments, some nucleotide sequences of the disclosure can be synthesized by artificial synthesis method under suitable conditions.

In this disclosure, the proteins of interest can be various proteins well known in the art, including but not limited to enzymes, antibodies, and other proteins with desired functions. Preferably, proteins of interest are proteins well-known in the art that need to be expressed in T cells, such as various antibodies with anti-tumor effect, including single-chain antibody, or chimeric antigen receptor (CAR).

The enhanced promoter sequence of the disclosure is particularly suitable for driving the expression of genes of various antibodies, preferably full-length antibodies, in T cells. Exemplary antibodies include but are not limited to a PD-1 antibody, a PD-L1 antibody, a CTLA4 antibody, a CD40 antibody, a CD40L antibody, a CD47 antibody, a CD137 antibody, a CD28 antibody, a CD27 antibody, a OX40 antibody, a DNAM-1 antibody, a GITR antibody, a ICSOS antibody, a 2B4 antibody, a CD160 antibody, a TIM3 antibody, a LAG3 antibody, a BTLA antibody and a TIGIT antibody.

In some embodiments, the expression vector of the disclosure comprises the enhanced promoter sequence described herein and a coding sequence of PD-1 antibody, which is used to express PD-1 antibody in T cells or to integrate the expression cassette of PD-1 antibody into the genome of T cells.

The vector of the disclosure can be transferred into cells of interest by conventional transfection methods, including but not limited to virus transduction, microinjection, particle bombardment, gene gun transformation and electroporation. In some embodiments, the vector described herein is transfected into the cells of interest by electroporation.

The cells of interest can be various T cells well known in the art, including but not limited to peripheral blood T lymphocytes, cytotoxic T cells (CTLs), helper T cells, inhibitory/regulatory T cells, γδT cells and cytokine-induced killer cells (CIK), tumor infiltrating lymphocytes (TIL), or T cells of mixed cell populations. In some embodiments, the T cells can be derived from PBMC of patients with B cell malignancies. In some embodiments, the T cells are primarily cultured T cells.

Therefore, in some embodiments, the disclosure provides the use of the enhanced promoter sequence described herein in driving the expression of exogenous gene(s) (such as full-length antibody genes) in T cells.

In some embodiments, the disclosure also provides a T cell comprising the enhanced promoter sequence or nucleic acid construct or vector described herein. Preferably, the genome of the T cell is integrated with an expression cassette, and the enhanced promoter sequence described herein is used in the expression cassette as a promoter to drive the expression of an exogenous gene of interest. More preferably, the genome of the T cell of the disclosure is integrated with an expression cassette comprising the enhanced promoter sequence described herein and the coding sequence of PD-1 antibody which is operably linked to the promoter sequence. In some embodiments, compared with the control T cells without the enhancer sequence described herein but containing the same promoter sequence, the expression amount of the protein of interest of the T cells according to the disclosure is at least 1.1 times, at least 1.2 times or at least 1.5 times of the expression amount of the protein of interest of the control T cells, as measured under the same conditions.

In some embodiments, the amino acid sequence of the PD-1 antibody described herein is as shown in SEQ ID NO: 11, and its exemplary coding sequence is as shown in SEQ ID NO: 10.

The embodiments of the present disclosure illustrated by way of specific examples below. Those skilled in the art will understand that these examples are merely exemplary and should not be considered as limiting the scope of the present disclosure. The experimental methods without specifying the specific technology or conditions in the following examples generally used the conventional technology or conditions, such as those described in J. Sambrook et al., Molecular Cloning: A Laboratory Manual (3rd ed.), translated by Huang Peitang et al., Science Press, or followed the manufacturer's recommendation. The used reagents or instruments without specifying the manufacturer are all conventional products that are commercially available.

### Example 1: Construction of expression vector carrying nucleotide sequence of each promoter

### 1. Construction of enhanced green fluorescent protein EGFP expression vector

Sequence sDTS-LTR (SEQ ID NO: 2) was synthesized by Shanghai Generay Biotech Co., Ltd with AscI restriction site introduced into its upstream and XbaI restriction site inserted into its downstream. The sequence was inserted into pNB328 vector (pNB328 vector comprises EF1α promoter; its structure and sequence are as described in CN201510638974.7, the whole content of which is incorporated herein by reference) which was double digested by AscI and *Xba*I, to construct the recombinant plasmid, named pNB338A-E.

Sequence sDTS (SEQ ID NO: 1) was synthesized by Shanghai Generay Biotech Co., Ltd with XbaI restriction site introduced into its upstream and PvuI restriction site inserted into its downstream. The sequence was inserted into pNB328 vector (which was double digested by XbaI and PvuI, to construct the recombinant plasmid, named pNB338B-E.

Green fluorescent protein EGFP gene sequence (its nucleic acid sequence is as shown in SEQ ID NO: 8, its amino acid sequence is as shown in SEQ ID NO: 9) was synthesized by Shanghai Generay Biotech Co., Ltd with EcoRI restriction site introduced into its upstream and SalI restriction site inserted into its downstream. The sequence was respectively inserted into pNB328 vector, pNB338A-E vector and pNB338B-E vector which were double digested by EcoRI and SalI, to construct the recombinant plasmids, named pNB328-E-EGFP, pNB338A-E-EGFP and pNB338B-E-EGFP, respectively.

### 2. Construction of luciferase reporter gene expression vector carrying each promoter

The coding sequence of nano luciferase (NLuc for short) gene (its nucleic acid sequence is as shown in SEQ ID NO: 6, its amino acid sequence is as shown in SEQ ID NO: 7) in pNL1.3.CMW[secNluc-CMV] plasmid was synthesized by Shanghai Generay Biotech Co., Ltd with EcoRI restriction site introduced into its upstream and SalI restriction site inserted into its downstream. The sequence was respectively inserted into pNB328-E vector and pNB338B-E vector which were double digested by EcoRI and SalI, to construct the recombinant plasmids, named pNB328-E-NLuc and pNB338B-E-NLuc, respectively.

The nucleotide sequences of hCMV promoter and β-Actin promoter as shown in SEQ ID NO: 4 and SEQ ID NO: 5 were synthesized by Shanghai Generay Biotech Co., Ltd with XbaI introduced into its upstream and EcoRI restriction site introduced into its downstream. The sequences were inserted into pNB328-E-NLuc vectors which were double digested by XbaI and EcoRI (replacing the EF1α promoter in the original pNB328-E-NLuc vector), to construct the recombinant plasmids, named pNB328-hC-NLuc and pNB328-β-NLuc, respectively. The sequences were also inserted into pNB338B-E-NLuc vectors which were double digested by XbaI and EcoRI, to construct the recombinant plasmids, named pNB338B-hC-NLuc and pNB338B-β-NLuc, respectively.

### 3. Construction of PD-1 antibody expression vector carrying each promoter

PD-1 antibody nucleotide sequence (its nucleic acid sequence is as shown in SEQ ID NO: 10, its amino acid sequence is as shown in SEQ ID NO: 11) was synthesized by Shanghai Generay Biotech Co., Ltd with EcoRI restriction site introduced into its upstream and SalI restriction site inserted into its downstream. The sequence was inserted into pNB328-E-NLuc, pNB328-hC-NLuc, pNB328-β-NLuc, pNB338B-E-NLuc, pNB338B-hC-NLuc and pNB338B-β-NLuc vectors which were double digested by EcoRI and SalI respectively, to construct the recombinant plasmids, named pNB328-E-m279V, pNB328-hC-m279V, pNB328-β-m279V, pNB338B-E-m279V, pNB338B-hC-m279V and pNB338B-m279V, respectively.

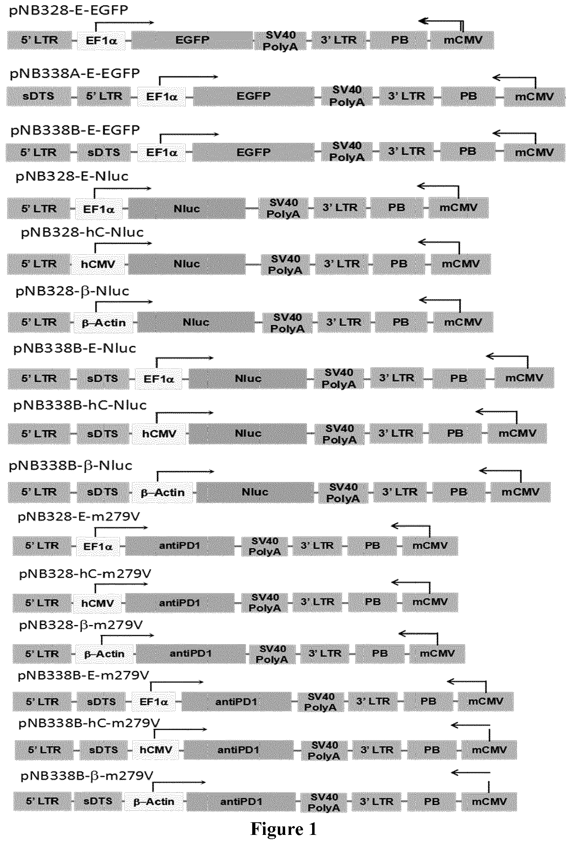

The schematic diagram of the structure of each expression vector is shown in Figure 1.

### Example 2: Construction of T cells

Peripheral blood mononuclear cells (PBMCs) were isolated from donor blood by Filcoll isolation method. PBMCs were cultured adherently for 2-4h, wherein the non-adherent suspension cells were the initial T cells. The suspension cells were collected into a 15 ml centrifuge tube and centrifuged at 1200 rmp for 3 min, the supernatant was discarded, normal saline was added, the mixture was centrifuged at 1200 rmp for 3 min, normal saline was discarded and the procedure was repeated.

15 of 1.5 ml centrifugation tubes with 5×10⁶ cells added into each tube were numbered 1-15 and centrifuged at 1200 rmp for 3min, the supernatant was discarded, 100 ul of the electroporation reagent from the Electroporation Kit (Lonza company) was added into each tube in proportion, and 6 ug of the above plasmids was added into 1-15 tubes respectively, and the cells were re-suspended; the mixed liquid was transferred to electroporation cuvettes and placed into the electroporator, the required program was selected and the electrical pulse was produced; the electroporated cell suspension was transferred to six-well plates supplemented with medium (AIM-V medium containing 2% FBS) using the micropipette in the kit, evenly mixed and cultured at 37°C, 5% CO₂ in an incubator, after 6 hours, stimulating factor IL-2 and CD3 antibody, CD28 antibody were added, after 3-4 days of culture at 37°C, 5% CO₂, the growth of T cells was observed, and T cells expressing EGFP, Nluc and PD-1 antibody were obtained.

### Example 3: Comparison and identification of EGFP expressed by pNB328-E-EGFP, pNB338A-E-EGFP and pNB338B-E-EGFP plasmids

The activated T cells modified with pNB328-E-EGFP, pNB338A-E-EGFP and pNB338B-E-EGFP plasmids obtained from Example 2 were observed under fluorescence microscope on the 8^{th} and 18^{th} days after electroporation, and 5×10⁵ cell precipitates were collected respectively and washed by PBS for 2 times, 400 ul PBS was added before transferring the cells into flow cytometry tubes, and the flow cytometry tubes were loaded onto the flow cytometry device for detection.

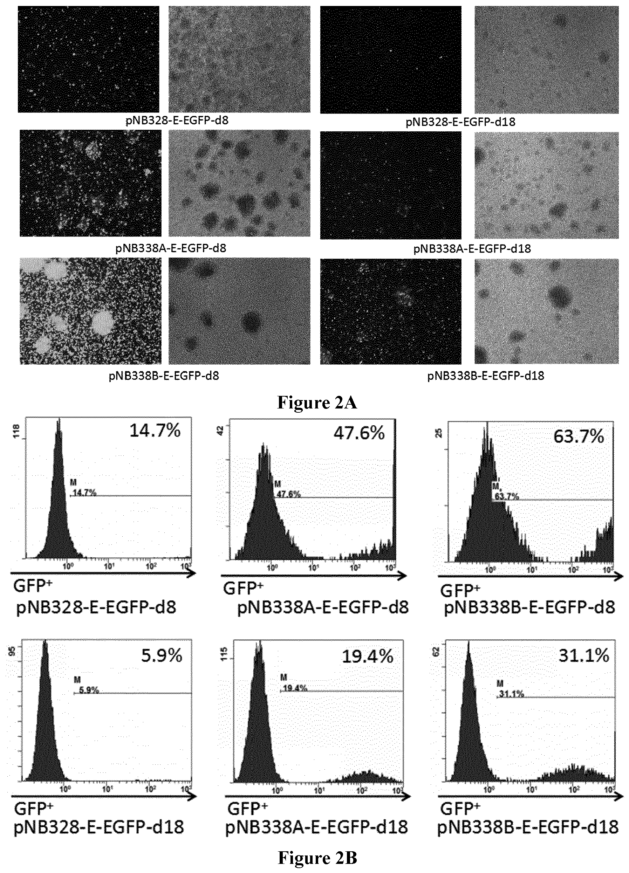

Results are shown in Figure 2A and 2B and the following Table 1, both the positive rate and fluorescence intensity of the expression of pNB338B-E-EGFP plasmid were better than those of pNB338A-E-EGFP plasmid, while the positive rate and fluorescence intensity of the expression of pNB338A-E-EGFP plasmid were better than those of pNB328-E-EGFP plasmid. This indicates that the expression of the plasmid containing sDTS sequence is stronger than that of the plasmid without sDTS sequence, and the expression of the plasmid in which sDTS sequence is located before EF1α promoter and after 5' LTR (pNB338B-E-EGFP) is stronger.

**Table 1**

| Cell type | Culture days | Fluorescence intensity |
|---|---|---|
| pNB328-E-EGFP T cell | **8** | **188** |
| pNB338A-E-EGFP T cell | **8** | **360** |
| pNB338B-E-EGFP T cell | **8** | **510** |
| pNB328-E-EGFP T cell | **18** | **76** |
| pNB338A-E-EGFP T cell | **18** | **179** |
| pNB338B-E-EGFP T cell | **18** | **318** |

### Example 4: Comparison of Nluc expressed by pNB328-E-Nluc, pNB338B-E-Nluc, pNB328-hC-Nluc, pNB338B-hC-Nluc, pNB328-β-Nluc and pNB338β-β-Nluc plasmids

2×10⁶ of each of the activated T cells modified with pNB328-E-Nluc, pNB338B-E-Nluc, pNB328-hC-Nluc, pNB338B-hC-Nluc, pNB328-β-Nluc and pNB338B-β-Nluc plasmids obtained from Example 2 were collected on the 7^{th} and 17^{th} days, placed into 6-well plates supplemented with 3 ml AIM-V medium at the density of 2×10⁶ cells/well, and incubated at 37°C, 5% CO₂ in an incubator. After 24 h of culture, the cell supernatant was collected and stored at -20°C for later use. The fluorescence value of the novel fluorescein Nluc secreted into the cell supernatant was measured on a Microplate Reader.

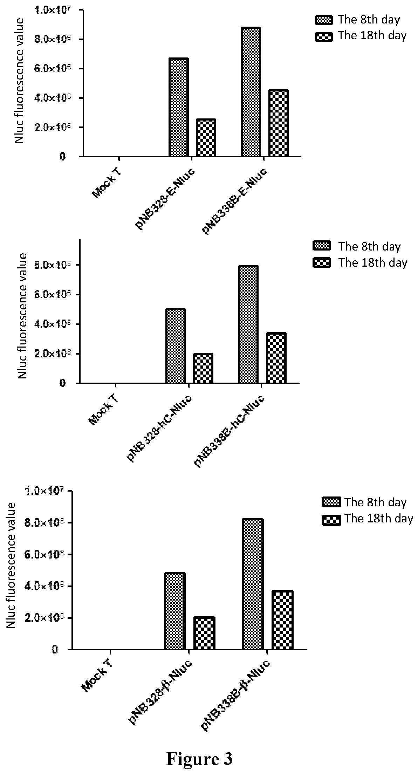

Results are shown in Figure 3 and the following Tables 2-4, for different promoters (EF1α/hCMV/β-Actin), all the Nluc fluorescence values expressed by the plasmids (pNB338B-E-Nluc, pNB338B-hC-Nluc, pNB338B-β-Nluc) containing sDTS sequence were higher than those of the plasmids (pNB328-E-Nluc, pNB328-hC-Nluc, pNB328-β-Nluc) with the same promoter but without sDTS sequence.

**Table 2**

| Cell type | Culture days | Nluc fluorescence value |
|---|---|---|
| Mock T cell | **8** | **286** |
| Mock T cell | **18** | **300** |
| pNB328-E-Nluc T cell | **8** | **6666082** |
| pNB328-E-Nluc T cell | **18** | **2528848** |
| pNB338B-E-Nluc T cell | **8** | **8772142** |
| pNB338B-E-Nluc T cell | **18** | **4511353** |

**Table 3**

| Cell type | Culture days | Nluc fluorescence value |
|---|---|---|
| Mock T cell | **8** | **300** |
| Mock T cell | **18** | **332** |
| pNB328-hC-Nluc T cell | **8** | **5056150** |
| pNB328-hC-Nluc T cell | **18** | **1984001** |
| pNB338B-hC-Nluc T cell | **8** | **7935584** |
| pNB338B-hC-Nluc T cell | **18** | **3382779** |

**Table 4**

| Cell type | Culture days | Nluc fluorescence value |
|---|---|---|
| Mock T cell | **8** | **293** |
| Mock T cell | **18** | **315** |
| pNB328-β-Nluc T cell | **8** | **4841137** |
| pNB328-β-Nluc T cell | **18** | **2028848** |
| pNB338B-β-Nluc T cell | **8** | **8235377** |
| pNB338B-β-Nluc T cell | **18** | **3680633** |

The results also indicate that, regardless of the expressed genes, all the expression plasmids containing sDTS sequence mediated by different promoters are better than the plasmids without sDTS sequence.

### Example 5: Comparison of the expression of PD-1 antibody expressed by pNB328-E-m279V, pNB338B-E-m279V, pNB328-hC-m279V, pNB338B-hC-m279V, pNB328-β-m279V and pNB338B-β-m279V plasmids

2×10⁶ of each of the activated T cells modified with pNB328-E-m279V, pNB338B-E-m279V, pNB328-hC-m279V, pNB338B-hC-m279V, pNB328-β-m279V and pNB338B-β-m279V plasmids obtained from Example 2 were collected on the 8^{th} and 18^{th} days, placed into 6-well plates supplemented with 3 ml AIM-V medium at the density of 2×10⁶ cells/well, and incubated at 37°C, 5% CO₂ in an incubator. After 24 h of culture, the cell supernatant was collected and stored at -20°C for later use. A double antibody sandwich ELISA method (using human PD-1 recombinant protein to coat ELISA plates, HRP-labeled mouse anti-human IgG4 mAb to detect, and commercialized anti-PD-1 antibody as standard) was used, and the sample to be tested was diluted by 50-folds before quantitative detection.

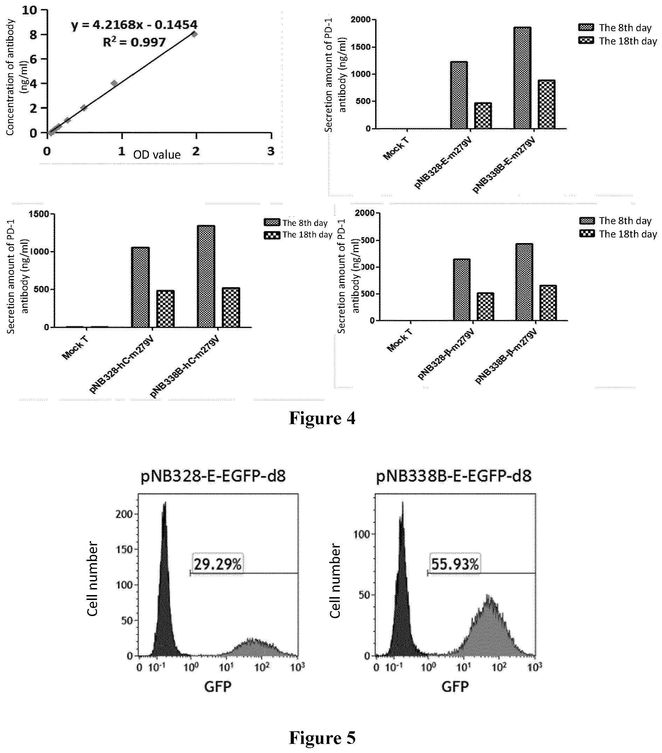

Results are shown in Figure 4 and the following Tables 5-7, for different promoters (EF1α/hCMV/β-Actin), all the amounts of the PD-1 antibody expressed by the plasmids (pNB338B-E-m279V, pNB338B-hC-m279V, pNB338B-β-m279V) containing sDTS sequence were higher than those of the plasmids (pNB338B-E-m279V, pNB338B-hC-m279V, pNB338B-β-m279V) with the same promoter but without sDTS sequence.

**Table 5**

| Cell type | Culture days | PD1 antibody (ng/ml) |
|---|---|---|
| Mock T cell | **8** | **0.23** |
| Mock T cell | **18** | **0.13** |
| pNB328-E-m279v T cell | **8** | **1230** |
| pNB328-E-m279v T cell | **18** | **470** |
| pNB338B-E-m279v T cell | **8** | **1860** |
| pNB338B-E-m279v T cell | **18** | **890** |

**Table 6**

| Cell type | Culture days | PD1 antibody (ng/ml) |
|---|---|---|
| Mock T cell | **8** | **1.51** |
| Mock T cell | **18** | **1.73** |
| pNB328-hC-m279v T cell | **8** | **1060** |
| pNB328-hC-m279v T cell | **18** | **488** |
| pNB338B-hC-m279v T cell | **8** | **1350** |
| pNB338B-hC-m279v T cell | **18** | **522** |

**Table 7**

| Cell type | Culture days | PD1 antibody (ng/ml) |
|---|---|---|
| Mock T cell | **8** | **0.44** |
| Mock T cell | **18** | **0.49** |
| pNB328-β-m279v T cell | **8** | **1145** |
| pNB328-β-m279v T cell | **18** | **513** |
| pNB338B-β-m279v T cell | **8** | **1437** |
| pNB338B-β-m279v T cell | **18** | **660** |

The results also indicate that all the expression plasmids containing sDTS sequence mediated by different promoters are better than the plasmids without sDTS sequence. Meanwhile, the plasmids containing sDTS sequence not only have better ability to express reporter genes, but also have stronger ability to express functional proteins.

### Comparative example 1: Expression of EGFP protein in CHO cells mediated by EFla and sDTS+EFla promoters

The pNB328-E-EGFP and pNB338B-E-EGFP obtained from Example 1 were used to transfect CHO cells respectively. Specifically, 2 centrifugation tubes were added with 5×10⁶ CHO cells each and centrifuged at 1200 rmp for 3min, the supernatant was discarded, 100 ul of the electroporation reagent from electroporation kit (Lonza company) was added into each tube in proportion, and 6 ug of pNB328-E-EGFP and 6 ug of pNB338B-E-EGFP were added into each tube respectively, and the cells were re-suspended respectively; the mixed liquid was transferred to electroporation cuvettes and placed into the electroporator, the required program was selected and the electric pulse was produced. After electroporation, CHO cells were cultured in 1640/DMEM (1:1 mixed) medium containing 10% FBS at 37°C, 5% CO₂ in an incubator. On the 8^{th} day after electroporation, precipitates of 5×10⁵ cell were collected and washed by PBS for 2 times, 400 ul PBS was added before transferring the cells into flow cytometry tubes, and the flow cytometry tubes were loaded onto the flow cytometry device for detection. Results are shown in Figure 5

On the 8^{th} day, the positive rate of the CHO cells transfected with pNB338B-E-EGFP was about 1.9 times of the positive rate of the CHO cells transfected with pNB328-E-EGFP. In Example 3, on the 8^{th} day, the positive rate of the T cells transfected with pNB338B-E-EGFP was about 4.3 times of the positive rate of the T cells transfected with pNB328-E-EGFP. This result indicates that the enhanced promoter of the disclosure can enhance the promoter transcription intensity specifically in T cells.

### Comparative example 2: Comparison of the expression of PD-1 antibody expressed by pNB338h-E-m279V, pNB338h-hC-m279V, pNB338h-β-m279V, pNB338e-E-m279V pNB338e-hC-m279V and pNB338e-β-m279V plasmids

The pNB338h-β-m279V, pNB338h-hC-m279V, pNB338h-β-m279V, pNB338e-β-m279V, pNB338e-hC-m279V and pNB338e-β-m279V recombinant plasmids were constructed according to the method of Example 1. Different from pNB338B-E-m279V, pNB338B-hC-m279V and pNB338B-β-m279V, hCMV enhancer was used in pNB338h-β-m279V, pNB338h-hC-m279V and pNB338h-β-m279V to replace sDTS sequence, and CD3e enhancer was used in pNB338e-β-m279V, pNB338e-hC-m279V and pNB338e-β-m279V to replace sDTS sequence.

2×10⁶ of each of the activated T cells modified with pNB338h-β-m279V, pNB338h-hC-m279V, pNB338h-β-m279V, pNB338e-β-m279V, pNB338e-hC-m279V and pNB338e-β-m279V plasmids were collected on the 8^{th} and 18^{th} days, placed into 6-well plates supplemented with 3 ml AIM-V medium at the density of 2×10⁶ cells/well, and incubated at 37°C, 5% CO₂ in an incubator. After 24 h of culture, the cell supernatant was collected and stored at -20°C for later use. A double antibody sandwich ELISA method (using human PD-1 recombinant protein to coat ELISA plates, HRP-labeled mouse anti-human IgG4 mAb to detect, and commercialized anti-PD-1 antibody as standard) was used, and the sample to be tested was diluted by 50-fold before quantitative detection.

Results show that, for different promoters (EF1α/hCMV/β-Actin), all the amounts of the PD-1 antibody expressed by the plasmids (pNB338B-E-m279V, pNB338B-hC-m279V, pNB338B-β-m279V) containing sDTS sequence were higher than those of the plasmids with the same promoter but containing hCMV enhancer sequence or CD3e enhancer sequence.

These results indicate that all the expression plasmids containing sDTS sequence mediated by different promoters are better than the plasmids containing other enhancer sequences. Meanwhile, the ability of the plasmid containing sDTS sequence to express a specific gene is stronger than the ability of other enhancer sequence to mediate gene expression.

## Claims

1. An enhanced promoter sequence, wherein the enhanced promoter sequence consists of an enhancer sDTS and a promoter operably linked to the enhancer, wherein the sequence of the enhancer sDTS is as shown in SEQ ID NO: 1; and the enhancer is located at a 5' end or a 3' end, preferably a 5' end, of the promoter.

2. The enhanced promoter sequence according to claim 1, wherein the promoter is selected from the group consisting of: an EF1α promoter, a PGK promoter, a β-actin promoter, a hCMV promoter, an EEF2 promoter, a CAG promoter, a U6 promoter and a SV40 promoter; preferably, the promoter is an EF1α promoter, a hCMV promoter or a β-actin promoter, more preferably, the promoter is an EF1α promoter;
preferably, the nucleotide sequence of the EF1α promoter is as shown in SEQ ID NO: 3; the nucleotide sequence of the hCMV promoter is as shown in SEQ ID NO: 4; the nucleotide sequence of the β-actin promoter is as shown in SEQ ID NO: 5.

3. A nucleic acid construct, wherein the nucleotide construct comprises the enhanced promoter sequence according to claim 1 or 2;
preferably, the nucleic acid construct is an expression cassette, which sequentially contains, from the 5' end to the 3' end, the enhanced promoter sequence, a coding sequence of a protein of interest and a transcription terminator, that are operably linked.

4. A vector, wherein the vector comprises the enhanced promoter sequence according to claim 1 or 2.

5. The vector according to claim 4, wherein the vector is a eukaryotic expression vector, selected from the group consisting of: a transient expression vector, a virus expression vector and a transposition vector;
preferably, the vector is a vector for integrating an expression cassette of a gene of interest into the genome of a host cell, preferably is a transposition vector; preferably, the transposition vector comprises a transposable element selected from the group consisting of: piggyback, sleeping beauty, frog prince, Tn5 and Ty.

6. The vector according to claim 5, wherein the transposition vector sequentially contains the enhanced promoter sequence, one or more restriction sites or optional coding sequence(s) of interest, and a transcription termination sequence between the 5' LTR and the 3' LTR, that are operably linked;
preferably, a coding sequence of a transposase and its promoter sequence are operably linked at the 3' end of the 3' LTR;
preferably, the nucleic acid sequence of the vector is as shown in SEQ ID NO: 12;
preferably, the transcription termination sequence is SV40 polyA transcription termination sequence.

7. The vector according to claim 5 or 6, wherein,
the gene of interest is the coding sequence of a PD-1 single-chain antibody; and/or
the restriction site is selected from the group consisting of: an AscI restriction site, an XbaI restriction site, a PvuI restriction site, an EcoRI restriction site and a SalI restriction site.

8. Use of the enhanced promoter sequence according to claim 1 or 2 in driving the expression of an exogenous gene in a T cell.

9. A T cell, wherein the T cell comprises the enhanced promoter sequence according to claim 1 or 2, the nucleic acid construct according to claim 3, or the vector according to any of claims 4-7;
preferably, the genome of the T cell is integrated with an expression cassette, and the enhanced promoter sequence according to claim 1 or 2 is used in the expression cassette as a promoter to drive the expression of an exogenous gene of interest.

10. A T cell stably expressing an antibody, wherein the genome of the T cell is integrated with an expression cassette comprising the enhanced promoter sequence according to claim 1 or 2 and a coding sequence of the antibody operably linked to the promoter sequence;
preferably, the antibody is selected from the group consisting of: a PD-1 antibody, a PD-L1 antibody, a CTLA4 antibody, a CD40 antibody, a CD40L antibody, a CD47 antibody, a CD137 antibody, a CD28 antibody, a CD27 antibody, a OX40 antibody, a DNAM-1 antibody, a GITR antibody, an ICSOS antibody, a 2B4 antibody, a CD160 antibody, a TIM3 antibody, a LAG3 antibody, a BTLA antibody and a TIGIT antibody;
more preferably, the antibody is a PD-1 antibody; preferably, the amino acid sequence of the PD-1 antibody is as shown in SEQ ID NO: 10, preferably, the coding sequence thereof is as shown in SEQ ID NO: 11.
